# EUROPÄISCHE PATENTANMELDUNG

(11) **EP 1 618 860 A1**
(43) Veröffentlichungstag der Anmeldung: **25.01.2006**
(21) Anmeldenummer: 05009303.8
(22) Anmeldetag: 28.04.2005
(51) Int. Cl.: A61F 13/15

(54) **Verpackung eines Hygieneartikels, insbesondere eines Menstrualtampons**

(30) Priorität: 24.07.2004 DE 102004036058
(71) Anmelder: HYSALMA Hygiene Sales + Marketing Gesellschaft mit beschränkter Haftung, 45481 Mühlheim an der Ruhr (DE)
(72) Erfinder: Schmidt, Lothar, 09569 Oederan (DE); Nennstiel, Martina, 46147 Oberhausen (DE)
(74) Vertreter: COHAUSZ DAWIDOWICZ HANNIG & SOZIEN

(57) **Zusammenfassung**

Die Erfindung betrifft eine Einzelverpackung eines länglichen Einzelproduktes wie eines Hygieneartikels, insbesondere eines Tampons für die Frauenhygiene mit einer Folie/einem Papier, die/das den Artikel außen eng und insbesondere luftdicht umschließt, wobei die Folie/das Papier eine Schwächungslinie aufweist, bei der die Schwächung kontinuierlich und frei von Löchem über die Länge der Schwächungslinie verläuft.

## Beschreibung

Die Erfindung betrifft eine Einzelverpackung eines länglichen Einzelproduktes wie eines Hygieneartikels, insbesondere eines Tampons für die Frauenhygiene mit einer Folie oder einem Papier, die/das den Artikel außen eng und insbesondere luftdicht umschließt.

Es ist bekannt, Tampons der Frauenhygiene luftdicht durch eine Folie oder durch Papier zu umschließen. Hierbei hat es sich gezeigt, dass diese schwer zu öffnen sind, auch wenn sie einen Aufreißstreifen aufweisen. Einzelverpackungen neuerer Art ohne zusätzliche Aufreißstreifen sind problematisch in Bezug auf die notwendige Luftdichtheit und benötigten oft zusätzliche abdichtende Mittel und Griffigkeit erhöhende Mittel, um das gegenläufige Verdrehen der beiden Verpackungsenden zu ermöglichen.

Aufgabe der Erfindung ist es, das Öffnen von Einzelverpackungen der eingangs genannten Art zu erleichtern und hierbei die Sicherheit einer hohen Luftdichtigkeit und Hygiene zu gewährleisten.

Diese Aufgabe wird erfindungsgemäß dadurch gelöst, dass die Folie/das Papier eine Schwächungslinie aufweist, bei der die Schwächung kontinuierlich und frei von Löchern über die gesamte Länge der Schwächungslinie verläuft.

Die Schwächungslinie einer solchen Einzelverpackung wird ohne mechanische Einwirkung erzeugt und ist damit dicht, da durch die gleichmäßige Zufuhr von Energie eine sehr gleichmäßige Schwächung im Bereich der Linie erreicht wird, ohne Undichtigkeiten insbesondere Löcher zu erzeugen. Es finden somit bei der Herstellung keine gewollten oder ungewollten Perforationen des Verpackungsmittels statt. Die Einzelverpackung kommt ohne zusätzliche Öffnungshilfe aus, so dass sie auch nicht vom Anwender gesucht und verwendet werden muss. Die beim Öffnen der Verpackung entstehenden Knack-Geräusche sind ein Indikator für eine dichte - und somit hygienische Verpackung.

Vorteilhafte Ausgestaltungen der Erfindung sind in den Unteransprüchen aufgeführt.

Ausführungsbeispiele der Erfindung sind in den Zeichnungen dargestellt und werden im Folgenden näher beschrieben. Es zeigen
Fig. 1 einen Wickel eines Folien-/Papierbandes mit längs angeordneter Schwächungslinie;
Fig. 2 einen zylindrisch gebogenen Folien-/Papierabschnitt mit einem Tampon vor dem Einstecken;
Fig. 3 den Folien-/Papierabschnitt um den Tampon mit geschlossenem Kopf und Boden;
Fig. 4 einen Folien-/Papierabschnitt mit längs angeordneter Schwächungslinie;
Fig. 5 einen Folien-/Papierabschnitt mit wellenförmiger Schwächungslinie;
Fig. 6 eine Folien-/Papierverpackung mit quer zur Tamponlängsachse verlaufender Schwächungslinie;
Fig. 7 die Verpackung nach Fig. 6 nach dem Knicken und Aufreißen;
Fig. 8 eine Folien-/Papierverpackung mit S- oder stufenförmiger Schwächungslinie;
Fig. 9 die Verpackung nach Fig. 8 nach dem Aufreißen.

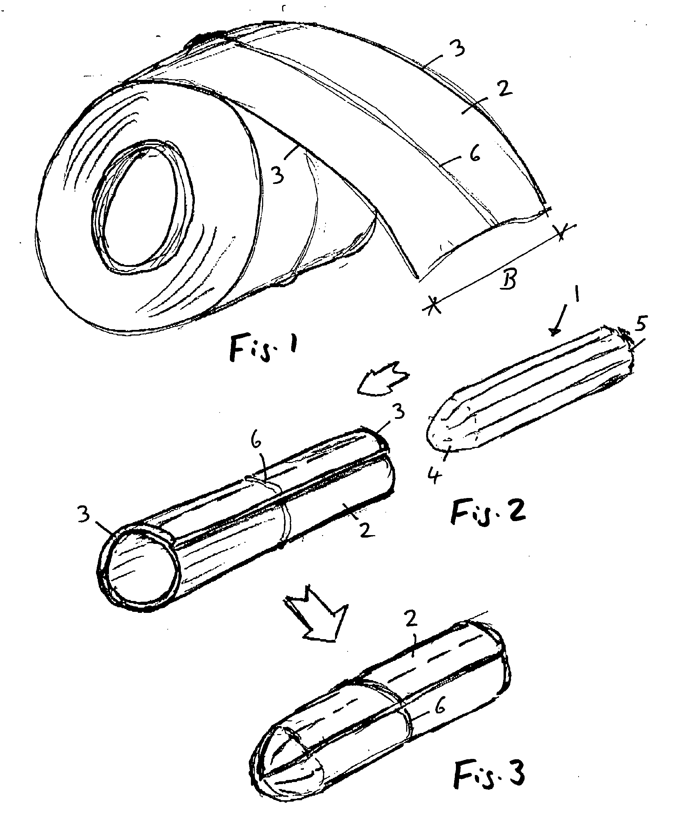

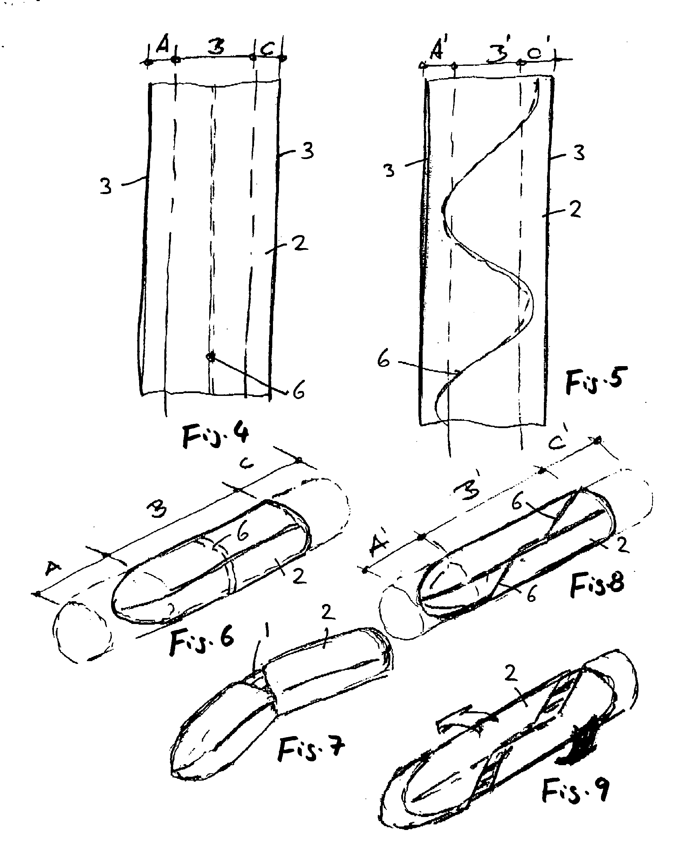

Um einen länglichen Gegenstand bzw. Artikel wie einen Tampon 1 zu verpacken, wird ein Folien-/Papierband 2 verwendet, das außen um den Gegenstand, insbesondere den Tampon 1 zylindrisch gewickelt wird. Hierbei wird die Breite B des Folien-/Papierbandes 2 so reichlich bemessen, dass nach dem Abschneiden eines Folien-/Papierabschnittes die Ränder 3 des Folien-/Papierbandes 2 um den Kopf 4 und den Boden 5 umgelegt und aneinander befestigt werden. Hierdurch entsteht eine luftdichte Einzelverpackung.

Das Folien-/Papierband 2 weist eine längs und damit im Wesentlichen parallel zu den Rändern 3 angeordnete Schwächungslinie 6 auf, die durch Materialschwächung hergestellt wurde. Aufgrund dieser Schwächungslinie 6 ist die Verpackung durch Kraftaufwendung, insbesondere durch Knicken leicht zu öffnen.

Es handelt sich um ein Verpackungsmaterial mit mindestens einer, im Bereich einer kontinuierlich verlaufenden Linie, eingebrachten Gefüge- /Strukturveränderung. Die Materialfestigkeit im Bereich dieser Linie fällt geringer aus, als in den Bereichen neben der Linie. Die gleichmäßige Schwächungslinie verläuft im Wesentlichen parallel zu den Seitenkanten des Verpackungsmaterials.

Die im Wesentlichen luftdichte, eng am Produkt anliegende, Einzelverpackung besteht aus bekannten Verpackungsmitteln (PP; PE; BOPP; Zellglas; Papier; Laminaten und artgleichen Verpackungsmitteln). In das Verpackungsmittel, insbesondere in diejenigen aus Kunststoffen, insbesondere BOPP (coextrudierte, biaxial gereckte und thermofixierte Polypropylenfolie) sind Schwächungslinien quer zur Verpackungslängsachse eingebracht. Im Bereich dieser Linien ist die Struktur / das Gefüge des Verpackungsmittels so verändert, dass durch Einwirkung einer gezielten Kraft auf die Verpackung, die Verpackung an dieser Stelle rückstandsfrei bricht, damit das Produkt entnommen werden kann.

Die Verpackung wird zum Öffnen quer zur Längsachse der Verpackung geknickt. Die so auf die Verpackung wirkende Kraft bewirkt, dass die Verpackung gegenüber der Knickstelle bricht. Je nach Grad der Knickung ist die Öffnung am Umfang der Verpackung unterschiedlich lang. Mehrmaliges Knicken an unterschiedlichen Stellen, Knicken in verschiedene Richtungen oder zusätzliches, gegenläufiges Verdrehen der beiden Verpackungsenden öffnet die Verpackung komplett.

Durch Zufuhr von Energie, vorzugsweise Lichtenergie (Laser) oder thermische Energie, sowie andere gezielt anwendbare Energieformen (Ultraschall, Radiowellen usw.) wird im Bereich der Schwächungslinie das Gefüge eines geeigneten Verpackungsmittels so verändert, dass es zumindest in einer elementaren Kraft- Einwirkungs- Art (Zug, Druck, Schub) eine deutliche Schwächung / eine erhöhte Empfindlichkeit gegenüber den Festigkeitseigenschaften des Ausgangsstoffes aufweist.

Beispielsweise gelingt es mittels geeignetem Laser, bei biaxial gereckter PP-Folie, die Festigkeitseigenschaften der Folie/des Papiers innerhalb einer durch den Laserstrahl bestrichenen Linie so gleichmäßig über die gesamte Länge der Linie zu verändern, dass die Folie/das Papier auf Zugkräfte, wie sie beim Knicken einer Tampon-Verpackung entstehen, aufbricht. Auf Druckkräfte und Schubkräfte zeigt der so veränderte Bereich deutlich geringere Empfindlichkeiten. Hierbei ist die Schwächung der Schwächungslinie über ihre Länge kontinuierlich und frei von Löchern, insbesondere von Perforierungen.

## Patentansprüche

1. Einzelverpackung eines länglichen Einzelproduktes wie eines Hygieneartikels, insbesondere eines Tampons (1) für die Frauenhygiene mit einer Folie/einem Papier (2), die/das den Artikel außen eng und insbesondere luftdicht umschließt, **dadurch gekennzeichnet, dass** die Folie/das Papier eine Schwächungslinie (6) aufweist, bei der die Schwächung kontinuierlich und frei von Löchern über die Länge der Schwächungslinie (6) verläuft.

2. Verpackung nach Anspruch 1, **dadurch gekennzeichnet, dass** die ohne mechanische Einwirkung erzeugte Schwächung der Schwächungslinie (6) eine gleichmäßige Struktur- und /oder Gefügeveränderung des Folien- /Papiermaterials ist.

3. Verpackung nach Anspruch 1 oder 2, **dadurch gekennzeichnet, dass** die Schwächung durch Zufuhr von Energie, insbesondere durch Strahlung wie Lichtenergie, insbesondere Laserstrahlung oder durch elektromagnetische Strahlung erfolgt.

4. Verpackung nach einem der vorstehenden Ansprüche, **dadurch gekennzeichnet, dass** die Schwächungslinie (6) im Wesentlichen quer oder schräg zur Artikellängsachse verläuft.

5. Verpackung nach einem der vorstehenden Ansprüche, **dadurch gekennzeichnet, dass** die Schwächungslinie (6) wellenförmig, stufenförmig und / oder in Zick-Zack-Form verläuft.

6. Verpackung nach einem der vorstehenden Ansprüche, **dadurch gekennzeichnet, dass** die Schwächungslinie (6) im Wesentlichen parallel zu den zwei Seitenkanten der Folien-/Papierbahn verläuft.

7. Verpackung nach einem der vorstehenden Ansprüche, **dadurch gekennzeichnet, dass** die Folie (2) aus Kunststoff, Zellglas, Metall, beschichtetem Papier und/oder einem Laminat besteht.

8. Verfahren zum Herstellen einer Verpackung nach einem der vorstehenden Ansprüche, **dadurch gekennzeichnet, dass** die Folie/das Papier (2) vor dem Umwickeln des Artikels insbesondere durch einen Laser mit der Schwächungslinie (6) versehen wird und dass danach der Artikel mit der Folie/dem Papier (2) umwickelt wird, wobei nach dem Umwickeln an beiden Enden des länglichen Artikels die Folie/das Papier geschlossen wird.

9. Verfahren zum Verwenden einer Verpackung nach einem der vorstehenden Ansprüche, **dadurch gekennzeichnet, dass** zum Öffnen der Verpackung diese quer zur Längsachse des länglichen Artikels so stark geknickt wird, bis die Verpackung an der Schwächungslinie (6) aufreißt.
